(19) Europäisches Patentamt
European Patent Office
Office européen des brevets

(11) Numéro de publication: **0 298 177 B1**

(12) # FASCICULE DE BREVET EUROPEEN

(45) Date de publication de fascicule du brevet: **15.04.92** (51) Int. Cl.[5]: **C07K 5/06**

(21) Numéro de dépôt: **87401587.8**

(22) Date de dépôt: **07.07.87**

Le dossier contient des informations techniques présentées postérieurement au dépôt de la demande et ne figurant pas dans le présent fascicule.

(54) **Nouveau procédé de préparation de dérivés de la pristinamycine IIB.**

(43) Date de publication de la demande:
**11.01.89 Bulletin 89/02**

(45) Mention de la délivrance du brevet:
**15.04.92 Bulletin 92/16**

(84) Etats contractants désignés:
**AT BE CH DE ES FR GB GR IT LI LU NL SE**

(56) Documents cités:
**EP-A- 0 135 410**
**EP-A- 0 191 662**
**EP-A- 0 252 720**

**R.J. Kennedy, J. Org. Chem. 25, p. 1901 (1960)**

**R. Bloch et coll., J. Org. Chem., 50, p. 1544 (1985)**

(73) Titulaire: **RHONE-POULENC SANTE**
**20, avenue Raymond Aron**
**F-92160 Antony(FR)**

(72) Inventeur: **Barrière, Jean-Claude**
**23 rue Henri Gilbert**
**F-91300 Massy(FR)**
Inventeur: **Bastart, Jean-Pierre**
**12 rue des Bergères Lésigny**
**F-77330 Ozoir La Ferrière(FR)**
Inventeur: **Paris, Jean-Marc**
**8 rue des Acacias**
**F-77360 Vaires Sur Marne(FR)**

(74) Mandataire: **Savina, Jacques et al**
**Rhône-Poulenc Rorer S.A. Direction Brevets**
**(t144) 20 avenue Raymond Aron**
**F-92165 Antony Cédex(FR)**

**EP 0 298 177 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention concerne un nouveau procédé de préparation de S-oxydes dérivés de la pristinamycine $II_B$.

La demande de brevet européen n° 191 662 décrit des S-oxydes dérivés de la pristinamycine $II_B$ de formule générale :

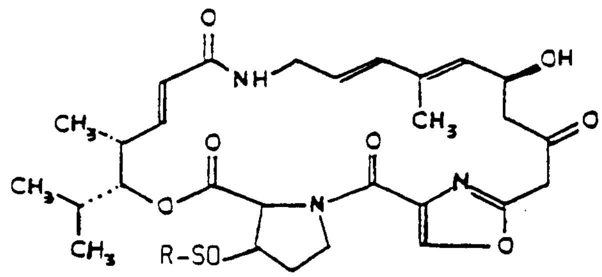

(I)

dans laquelle le symbole R représente

- soit un radical hétérocyclyle azoté de 4 à 7 chaînons, contenant éventuellement 1 ou plusieurs autres hétéroatomes choisis parmi l'azote, l'oxygène ou le soufre à l'état de sulfoxyde ou de sulfone, et éventuellement substitué par un radical alcoyle,
- soit une chaîne alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles ont rattachées un hétérocycle saturé ou insaturé de 4 à 7 chaînons contenant éventuellement un autre hétéroatome choisi parmi l'azote, l'oxygène ou le soufre à l'état de sulfoxyde ou de sulfone, et éventuellement substitué par un radical alcoyle), ou substituée par un ou plusieurs hétérocycles azotés de 4 à 7 chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins des substituants portés par la chaîne alcoyle ci-dessus est un substituant azoté capable de former des sels,
- soit un radical [méthyl-1 pyrrolidinyl-2(S)] méthyle, les radicaux alcoyle cités étant droits ou ramifiés et contenant, sauf mention spéciale, 1 à 10 atomes de carbone, leurs formes isomères ou leurs mélange, ainsi que leurs sels d'addition avec les acides et leur préparation.

Dans la formule générale (I) :

lorsque R représente un radical hétérocyclyle, ce radical peut être choisi à titre d'exemple parmi : azétidinyle-3, pyrrolidinyle-3, pipéridyle-3 ou -4 ou azépinyle-3 ou -4,

lorsque R représente un radical hétérocyclylalcoyle, le radical hétérocyclyle peut être choisi à titre d'exemple parmi les radicaux cités ci-dessus ou les radicaux azétidinyle-2, pyrrolidinyle-2, pipéridyle-2, azépinyle-2, pipérazinyle, alcoyl-4 pipérazinyle, quinolyle, isoquinolyle ou imidazolyle,

lorsque R contient un radical dialcoylamino ou dialcoylcarbamoyloxy dont les parties alcoyle forment avec l'atome d'azote auquel elles sont rattachées un hétérocycle, ce dernier peut être choisi à titre d'exemple parmi : azétidinyle-1, pyrrolidinyle-1, pipéridino, azépinyle-1, morpholino, thiomorpholino à l'état de sulfoxyde ou de sulfone, pipérazinyle-1, alcoyl-4 pipérazinyle-1, N-alcoyl homopipérazinyle-1, imidazolyle-1.

Il a maintenant été trouvé que les produits de formule générale (I) peuvent être obtenus par oxydation au moyen de monopersulfate de potassium, d'un dérivé de pristinamycine $II_B$, de son sel ou d'un dérivé protégé, de formule générale :

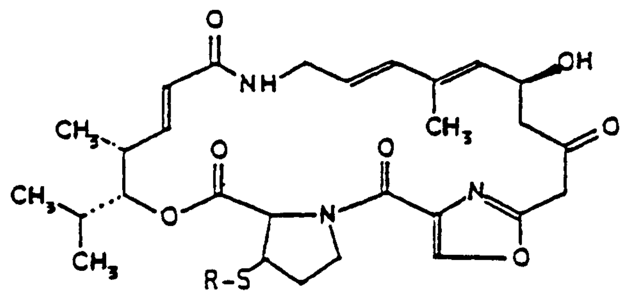

(II)

dans laquelle R est défini comme précédemment, étant entendu que dans les cas où R contient un hétérocycle soufré, l'atome de soufre peut se trouver à l'état de sulfure, de sulfoxyde ou de sulfone, puis éventuellement séparation des isomères obtenus.

On entend par monopersulfate de potassium le mélange contenant en outre du sulfate de potassium et du sulfate acide de potassium, tel que défini dans le brevet US 2 802 722 et commercialisé sous le nom d'Oxone®.

La réaction s'effectue dans l'eau ou dans un mélange eau-alcool (par exemple eau-méthanol) ou eau-solvant chloré (par exemple eau-dichlorométhane) à des températures comprises entre -60 et 25 °C.

Lorsque l'on met en oeuvre le dérivé de pristinamycine $II_B$ de formule générale (II) sous forme de sel, on utilise les sels formés avec les acides organiques ou minéraux, de préférence avec les acides trifluoracétique, tartrique, acétique, benzoïque chlorhydrique ou sulfurique ou avec l'hydrogénosulfate de potassium..

Lorsque R contient un substituant alcoylamino ou cycloalcoylamino, il est également possible de mettre en oeuvre un dérivé protégé du produit de formule générale (II), ce dernier peut être protégé par tout groupement protecteur d'amine dont la mise en place et l'élimination n'affectent pas le reste de la molécule ; on utilise avantageusement le groupement trifluoracétyle qui peut être éliminé après la réaction par traitement par un bicarbonate alcalin (bicarbonate de sodium ou de potassium) en solution aqueuse.

Le monopersulfate de potassium est préparé selon la méthode décrite dans le brevet US 2 802 722.

Les produits de formule générale (II) peuvent être obtenus selon la méthode décrite dans la demande de brevet européen n° 191 662. Il n'est pas absolument nécessaire d'isoler le dérivé de la pristinamycine $II_B$ de formule générale (II) pour mettre en oeuvre le procédé selon l'invention.

Les produits de formule générale (I) obtenus à l'issue de ce procédé, peuvent être purifiés par les méthodes connues, par exemple par cristallisation, chromatographie ou extractions successives en milieu acide ou basique. Pour l'homme de métier connaissant la sensiblité des synergistines en milieu alcalin on entend par "milieu basique" un milieu juste suffisamment alcalin pour libérer la substance mère de son sel d'addition avec un acide c'est-à-dire un milieu dont le pH n'excède pas 8.

Les isomères des produits de formule générale (I) peuvent être séparés par toute méthode connue. On opère avantageusement par chromatographie ou par chromatographie liquide hautes performances.

Les produits de formule générale (I) obtenus par le procédé selon l'invention manifestent une activité antibactérienne sur les bactéries gram-positives (du genre staphylocoques, streptocoques, pneumocoques, entérocoques) et gram-négatives (du genre haemophilus, gnocoques, méningocoques). De plus ils présentent l'avantage de pouvoir être solubilisés dans l'eau, généralement à l'état de sels, aux doses thérapeutiques utilisables et d'exalter par un phénomène de synergie l'action antibactérienne de la pristinamycine $I_A$, de la virginiamycine S ou de dérivés solubles tels que décrits dans la demande de brevet européen n° 191 662.

De plus les produits de formule générale (I) obtenus par le procédé selon l'invention peuvent être utiles pour la préparation de sulfones dérivées de la pristinamycine $II_B$, de formule générale :

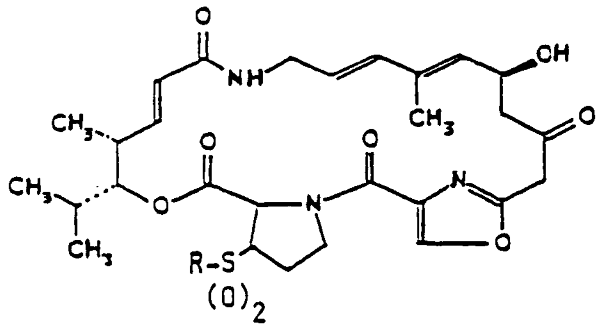

(III)

dans laquelle R est défini comme pour la formule générale (I), qui sont actives comme agents antimicrobiens et ont également la propriété de synergiser l'action des pristinamycines du group I.

La préparation des sulfones dérivées de la pristinamycine $II_B$ de formule générale (III) s'effectue par oxydation d'un dérivé de la pristinamycine $II_B$ de formule générale (I), dans les conditions décrites dans la demande de brevet européen n° 191 662.

Le procédé selon l'invention présente l'avantage d'être plus facilement réalisable et de permettre l'obtention d'un produit plus pur, le cas échéant plus facile à traiter dans la phase ultérieure de préparation de la sulfone correspondante, ou d'offrir moins de risques de dégradation du produit de départ et surtout moins de risques dans la mise en oeuvre de la préparation des produits de formule générale (I).

Plus particulièrement le procédé selon l'invention permet d'obtenir les produits de formule générale (I) pour lesquels le symbole R représente une chaîne alcoyle contenant 2 à 4 atomes de carbone substituée par 1 ou 2 radicaux choisis parmi phényle, cycloalcoyIamino ou N-alcoyl N-cycloalcoyl-amino contenant 5 ou 6 chaînons, alcoylamino contenant 1 à 4 atomes de carbone, dialcoylamino (dont les parties alcoyle contiennent 1 à 3 atomes de carbone ou forment avec l'atome d'azote auquel elles sont rattachées un hétérocycle saturé à 5 ou 6 chaînons), ou représente un hétérocycle azoté à 5 ou 6 chaînons éventuellement substitué par un radical alcoyle contenant 1 à 4 atomes de carbone, étant entendu que l'un au moins des substituants portés par la chaîne alcoyle ci-dessus est un substituant azoté capable de former des sels et que l'un au moins des radicaux portés par cette chaîne est placé en position-1 ou en position -2.

Les produits de formule générale (I) ainsi obtenus peuvent être transformés en sels.

Comme sels pharmaceutiquement acceptables, peuvent être cités notamment les sels d'addition avec les acides minéraux tels que chlorhydrates, bromhydrates, sulfates, nitrates, phosphates, ou organiques tels que acétates, propionates, succinates, maléates, fumarates, méthanesulfonates, p.toluènesulfonates, iséthionates, citrates, tartrates ou des dérivés de substitution de ces composés.

Les exemples suivants, donnés à titre non limitatif, montrent comment l'invention peut être mise en pratique. Les spectres de RMN des produits illustrés dans ces exemples présentent des caractéristiques générales qui sont communes à tous les produits de formule générale (I) et des caractéristiques particulières qui sont propres à chacun des produits en fonction des substituants. Dans les exemples ne sont mentionnées que les caractéristiques particulières dues aux radicaux variables. Pour les produits de formule générale (I) tous les protons sont désignés selon la numérotation indiquée dans la formule suivante :

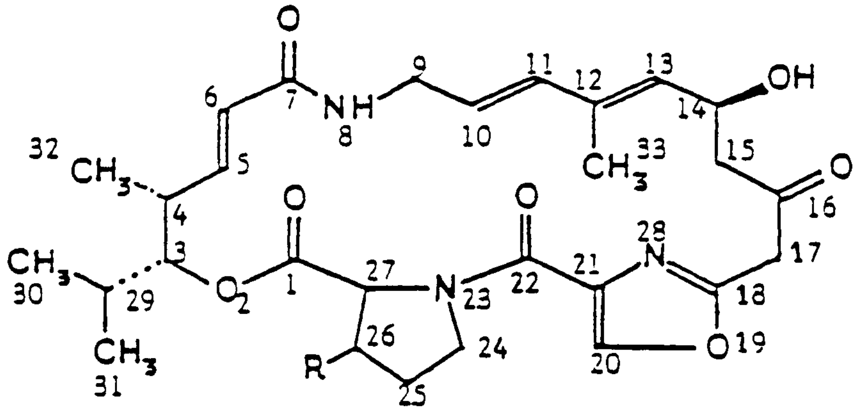

Sauf mention spéciale, tous les spectres ont été faits à 250 MHz dans le deutérochloroforme ; les déplacements chimiques sont exprimés en ppm par rapport au signal du tétraméthylsilane. Les abréviations utilisées dans la suite sont les suivantes :

s = singulet
d = doublet
t = triplet
mt = multiplet
m = massif
dd = doublet de doublet
dt = doublet de triplet
ddd = doublet de doublet de doublet
dddd = doublet de doublet de doublet de doublet

Il est entendu que les différents isomères ont été classés arbitrairement selon les déplacements chimiques observés en RMN.

On appelle isomère $A_1$ et isomère $A_2$ des produits de formule générale (I), les isomères qui présentent les caractéristiques :
environ 1,7 (s, $-CH_3$ en 33) ; environ 3,8 (s, $>CH_2$ en 17) ; <5 (d, $-H_{27}$) isomère $A_2$ ou >5 (d, $-H_{27}$) isomère $A_1$ ; environ 5,50 (d large, $-H_{13}$) ; environ 6,20 (d, $-H_{11}$) ; environ 6,6 ( $>NH$ en 8) ; ≧8 (s, $-H_{20}$).

On appelle isomère $B_1$ et isomère $B_2$ des produits de formule générale (I), les isomères qui présentent les caractéristiques :
environ 1,5 (s, $-CH_3$ en 33) ; environ 3,7 et 3,9 (2d, $>CH_2$ en 17) ; environ 4,8 (mt, $-H_{13}$) ; <5 (d, $-H_{27}$) isomère $B_2$ ou >5 (d, $-H_{27}$) isomère $B_1$ ; environ 5,70 (AB limite, $-H_{11}$ et $-H_{10}$) ; environ 7,7 ( $>NH$ en 8) ; environ 7,8 (s, $-H_{20}$).

On appelle isomère A du produit de formule générale (II) l'isomère qui présente des caractéristiques RMN identiques à celles énoncées ci-dessus pour les isomères $A_1$ et $A_2$ des produits de formule générale (I), étant entendu que l'H en 27 est caractérisé par : 4,7 (d, J ≦ 1 Hz).

On appelle isomère B du produit de formule générale (II) l'isomère qui présente des caractéristiques RMN identiques à celles énoncées ci-dessus pour les isomères $B_1$ et $B_2$ des produits de formule générale (I), étant entendu que l'H en 27 est caractérisé par : 4,6 (d, J ≧ 2,5 Hz).

## EXEMPLE 1

A 13 g de (diéthylamino-2 éthyl) thio-26 pristinamycine $II_B$ (isomère A) en suspension dans 170 cm3 d'eau distillée, on ajoute en 15 minutes à 0 ° C, 40 cm3 d'une solution aqueuse de 8,1 g d'Oxone® . Le mélange obtenu est agité 30 minutes à 0 ° C, puis additionné de 1,3 g de noir NORIT SX ULTRA et d'une petite quantité de thiosulfate de sodium. Après 30 minutes d'agitation à 20 ° C, la suspension est filtrée sur Celite puis rincée avec 50 cm3 d'eau distillée. La solution est ajustée à pH 7 par addition de bicarbonate de sodium solide puis lavée par 3 fois 100 cm3 de dichlorométhane. Les phases organiques sont réunies, séchées sur sulfate de magnésium, filtrées puis concentrées à sec sous pression réduite (2,7 kPa) à 30 ° C pour donner 9,9 g d'un solide beige clair contenant 85 % de (diéthylamino-2 éthyl) sulfinyl-26 pristinamycine $II_B$ (isomère $A_2$), 10 % de l'isomère $A_1$ et 5 % de (diéthylamino-2 éthyl) sulfonyl-26 pristinamycine $II_B$.

La forme cristalline de la (diéthylamino-2 éthyl) sulfinyl-26 pristinamycine $II_B$ peut être obtenue en opérant de la manière suivante :

5 g du solide obtenu précédemment sont repris dans 13 cm3 d'acétonitrile. Après dissolution à chaud, 10 cm3 d'éther sont ajoutés et la cristallisation est amorcée par grattage. Les cristaux sont filtrés, lavés à l'éther puis séchés sous pression réduite (270 Pa) à 20 ° C. On obtient ainsi 3,9 g de (diéthylamino-2 éthyl) sulfinyl-26 pristinamycine $II_B$ (isomère $A_2$ : 85 %, isomère $A_1$ : 15 %) sous forme de cristaux blancs fondant vers 116 ° C.

Spectre RMN (isomère $A_2$)
1,03 (t, $-N(CH_2\underline{CH_3})_2$)
1,75 (s, $-CH_3$ en $\overline{33}$)
2,05 et 2,55 (2mt, $>CH_2$ en 25)
2,45 à 2,70 (mt, $-N(\underline{CH_2}CH_3)_2$)

$$2,70 \text{ à } 3,10 \text{ (mt, } -\underset{\underset{O}{\downarrow}}{S}\underline{CH_2}-\underline{CH_2}-)$$

2,75 (mt, H en 4)
2,92 à 3,10 (mt, > $CH_2$ en 15)
3,22 (mt, H en 26)
3,82 (s, > $CH_2$ en 17)
4,81 (d, H en 27)
5,5 (d, H en 13)
6,19 (d, H en 11)
6,50 (dd, > NH en 8)
6,58 (dd, H en 5)
8,12 (s, H en 20)
Spectre RMN (isomère $A_1$)
1,04 (t, $-N(CH_2\underline{CH_3})_2$)
1,69 (s, $-CH_3$ en 33)
2 à 2,3 (mt, > $CH_2$ en 25)
2,60 (mt, > $N-CH_2-CH_3$)
2,7 à 2,95 (mt, $-S(O)-CH_2-CH_2-N$< )
2,7 (mt, H en 4)
2,86 et 3,04 (2dd, > $CH_2$ en 15)
3,28 (mt, H en 26)
3,78 (système AB, > $CH_2$ en 17)
5,25 (d, H en 27)
5,4 (d, H en 13)
6,15 (d, H en 11)
6,60 (dd, H en 5)
6,83 (dd, > NH en 8)
8,08 (s, H en 20)

La (diéthylamino-2 éthyl) thio-26 pristinamycine $II_B$ peut être préparée comme décrit dans le brevet US 4 590 004.

## EXEMPLE 2

A 3 g de (diéthylamino-2 éthyl) thio-26 pristinamycine $II_B$ (isomère A) en suspension dans un mélange de 30 cm3 d'eau distillée et de 6 cm3 d'éthanol, on ajoute en 10 minutes à -5 °C, 10 cm3 d'une solution aqueuse de 1,9 g d'Oxone®. Après 10 minutes d'agitation, le mélange réactionnel est lavé par 2 fois 20 cm3 de dichlorométhane. La phase aqueuse est ajustée à pH 7 par addition de bicarbonate de sodium solide puis extraite par 4 fois 50 cm3 de dichlorométhane. Les phases organiques sont réunies, séchées sur sulfate de magnésium, filtrées puis concentrées à sec sous pression réduite (2,7 kPa) à 40 °C. On obtient ainsi 2,4 g d'un solide blanc contenant la (diéthylamino-2 éthyl) sulfinyl-26 pristinamycine $II_B$ (isomère $A_2$ : 88 %, isomère $A_1$ : 6 %) et 6 % de (diéthylamino-2 éthyl) sulfonyl-26 pristinamycine $II_B$. Les caractéristiques RMN du produit obtenu sont identiques à celles du produit de l'exemple 1.

## EXEMPLE 3

A 3,2 g de [diéthylamino-1 propyl-2(R)] thio-26 pristinamycine $II_B$ (isomère A : 87 %, B : 13 %) en solution dans 48 cm3 de méthanol, on ajoute lentement à -30 °C 1,65 g d'Oxone® en solution dans 32 cm3 d'eau distillée. Après 30 minutes à -30 °C puis 15 minutes à -40 °C on ajoute 30 cm3 d'eau distillée puis 3 g de noir NORIT SX ULTRA. Le mélange est agité 30 minutes à 20 °C, filtré sur Celite, puis rincé par 50 cm3 d'eau distillée. La phase aqueuse est lavée par 2 fois 100 cm3 d'acétate d'éthyle puis 50 cm3 d'éther éthylique avant d'être ajustée à pH 7 per addition de bicarbonate de sodium solide puis extraite par 2 fois 250 cm3 de dichlorométhane. Les phases organiques sont lavées par 50 cm3 d'eau distillée, séchées sur sulfate de magnésium, filtrées puis concentrées à sec sous pression réduite (2,7 kPa) à 30 °C. On obtient ainsi 2,4 g d'une meringue jaune très clair qui est agitée dans 40 cm3 d'un mélange pentane-éther diéthylique (50-50 en volumes). Après filtration puis séchage sous pression réduite (270 Pa) à 20 °C, on obtient 2,2 g de [diéthylamino-1 propyl-2(R)] sulfinyl-26 pristinamycine $II_B$ (isomère $A_2$ : 80 %) sous forme d'un solide blanc cassé fondant vers 140 °C.
Spectre RMN :
1,02 (t, $-N(CH_2-\underline{CH_3})_2$)

1,34 (d, 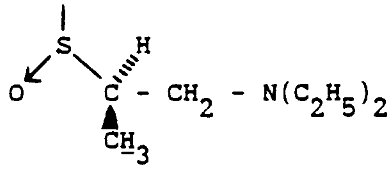 )

1,72 (s, $-CH_3$ en 33)
2,01 et 2,55 (2mt, > $CH_2$ en 25)
2,45 à 2,70 (mt, 1H de $-CH_2-N(CH_2-CH_3)_2$ et $-N(CH_2-CH_3)_2$)
2,90 (mt, 1H de $-CH_2-N(CH_2CH_3)_2$)
2,76 (mt, H en 4)
2,88 et 3,08 (2dd, > $CH_2$ en 15)

3 (mt, 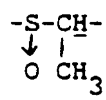 )

3,73 (mt, H en 26)
3,80 (s, > $CH_2$ en 17)
4,92 (s large, H en 27)
5,42 (d, H en 13)
6,15 (d, H en 11)
6,55 (dd, H en 5)
6,70 (dd, > NH en 8)
8,06 (s, H en 20)

La [diéthylamino-1 propyl-2(R)] thio-26 pristinamycine $II_B$ peut être obtenue de la façon suivante :

A 10,5 g de pristinamycine $II_A$ en suspension dans 200 cm3 de méthanol on ajoute à -30°C sous azote, 3,2 cm3 de diéthylamino-1 propanethiol-2(R). Après 18 heures d'agitation à -30°C, on ajoute 2 cm3 d'acrylate de méthyle et on agite 1 heure. On ajoute ensuite 200 cm3 d'eau distillée, du sulfate acide de potassium jusqu'à pH 4 à -10°C puis 10 g de noir NORIT SX ULTRA. Après 30 minutes d'agitation à 0°C le mélange est filtré sur Celite puis lavé par 150 cm3 d'eau distillée. La phase aqueuse est lavée par 100 cm3 d'acétate d'éthyle puis 100 cm3 d'éther éthylique puis ajustée à pH 7 par addition de bicarbonate de sodium solide. Après extraction par 200 cm3 de dichlorométhane, la phase organique est lavée par 100 cm3 d'eau, séchée sur sulfate de magnésium, filtrée puis concentrée à sec sous pression réduite (2,7 kPa) à 30°C. Le solide obtenu est trituré dans 100 cm3 d'éthylique, filtré sur verre fritté puis séché sous pression réduite (270 Pa) à 20°C. On obtient ainsi 3,36 g de [diéthylamino-1 propyl-2(R)] thio-26 pristinamycine $II_B$ (isomère A : 85 %, isomère B : 15 %) sous forme d'un solide beige clair fondant vers 130°C.

Spectre RMN (isomère A)
1,03 (t, $-N(CH_2-CH_3)_2$)

1,32 (d, 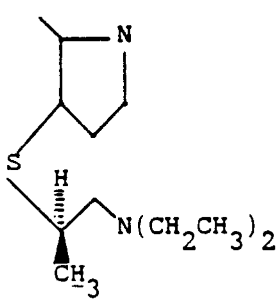 )

1,72 (s, $-CH_3$ en 33)
1,88 et 2,10 (2mt, > $CH_2$ en 25)
2,55 (mt, $-N(CH_2CH_3)_2$)
2,45 et 2,60 (2mt, $-CH_2-N(CH_2CH_3)_2$)

2,78 (mt, H en 4)
2,92 et 3,10 (2dd, $>CH_2$ en 15)

3 (mt, 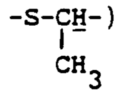

3,52 (mt, H en 26)
3,82 (s, $>CH_2$ en 17)
4,79 (s large, H en 27)
5,49 (d, H en 13)
6,13 (d, H en 11)
6,32 (m, $>NH$ en 8)
6,52 (dd, H en 5)
8,13 (s, H en 20)
(isomère B)
1 (t, $-N(CH_2CH_3)_2$)

1,34 (d, 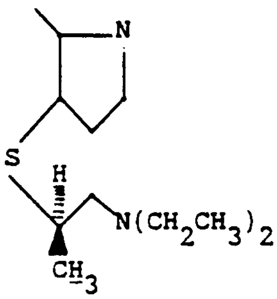 )

1,50 (s, $-CH_3$ en 33)
2,05 et 2,5 (2mt, $>CH_2$ en 25)
2,5 (mt, $>N-CH_2-CH_3$)
2,44 et 2,55 (2mt, $-CH_2-N(CH_2CH_3)_2$)
2,62 (mt, H en 4)
2,74 et 3,10 (2dd, $>CH_2$ en 15)

3,01 (mt, 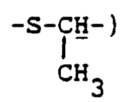

3,69 et 3,89 (2d, $>CH_2$ en 17)
3,74 (mt, H en 26)
4,01 (d, J = 2,5, H en 27)
4,80 (système AB, H en 13 et H en 14)
5,65 (d, H en 11)
6,60 (dd, H en 5)
7,71 (mt, $>NH$ en 8)
7,80 (s, H en 20)

Le diéthylamino-1 propanethiol-2(R) peut être préparé de la manière suivante :

A une suspension de 34,2 g d'hydrure de lithium et d'aluminium dans 1600 cm3 d'éther éthylique, on ajoute goutte à goutte en 1 heure et 20 minutes 137,5 g de N,N-diéthyl mercapto-2 propionamide(R) en solution dans 500 cm3 d'éther éthylique. Le mélange réactionnel est ensuite maintenu 2 heures et 30 minutes au reflux puis refroidi à une température voisine de 0 °C. On additionne alors 40 cm3 d'eau distillée de manière que la température du mélange ne dépasse pas 20 °C, puis 29,4 cm3 de soude 5N, puis 133 cm3 d'eau distillée. Le mélange réactionnel est filtré, le pH du filtrat est ajusté à 8 par addition de 70 cm3 d'acide acétique. Le mélange obtenu est à nouveau filtré, rincé par 3 fois 300 cm3 d'éther

éthylique, puis le filtrat est séché sur du sulfate de sodium, filtré et concentré à sec sous pression réduite (2,7 kPa) à une température voisine de 30 ° C. On obtient une huile jaune qui est purifiée par distillation sous pression réduite (1,3 kPa). On obtient ainsi 101 g de diéthylamino-1 propanethiol-2(R) sous forme d'une huile incolore [P.E.$_{1,3\ kPa}$ = 55-56 ° C ; $[\alpha]_D^{20}$ = -37,1 ° (c = 4,8, $CH_3OH$)].

Le N,N-diéthyl mercapto-2 propionamide(R) peut être préparé de la manière suivante :

A 517 cm3 d'une solution 5N de soude maintenue à 20 ° C, on ajoute en 30 minutes, 105 g de N,N-diéthyl acéthylthio-2 propionamide(R) dans 600 cm3 d'éther éthylique. le mélange réactionnel est agité 2 heures 30 minutes à une température voisine de 20 ° C. La phase aqueuse est séparée par décantation.

Le pH de la phase aqueuse est ensuite ajusté à pH = 5-6 par addition lente de 140 cm3 d'acide acétique. Le mélange obtenu est extrait par 300 cm3 de dichlorométhane puis 200 cm3 de dichlorométhane, les phases organiques réunies sont séchées sur du sulfate de sodium, filtrées puis concentrées à sec sous pression réduite (2,7 kPa) à une température voisine de 30 ° C. On obtient ainsi 78,6 g de N,N-diéthyl mercapto-2 propionamide(R) sous forme d'une huile violacée ($[\alpha]_D^{20}$ = -21,1 ° (c = 3,8 ; $CH_3$ OH) ).

Le N,N-diéthyl acétylthio-2 propionamide(R) peut être préparé de la manière suivante :

A une suspension de 72,6 g de sel de potassium de l'acide thiolacétique dans 300 cm3 d'éthanol, on ajoute 88,3 g de N,N-diéthyl chloro-2 propionamide(S) en solution dans 150 cm3 d'éthanol. Le mélange réactionnel est ensuite chauffé pendant 2 heures à une température voisine de 60 ° C, puis filtré et concentré a sec sous pression réduite (130 Pa) à une température voisine de 50 ° C. Le résidu obtenu est repris et agité dans 500 cm3 de dichlorométhane puis lavé avec 300 cm3 d'eau distillée, 300 cm3 d'une solution aqueuse de bicarbonate de potassium à 10 % puis 300 cm3 d'eau distillée. La phase organique est décantée, séchée sur du sulfate de sodium et 1 g de noir 3S, filtrée puis concentrée à sec sous pression réduite (2,7 kPa) à une température voisine de 30 ° C. Le résidu obtenu est distillé sous pression réduite (17 Pa). On obtient ainsi 105,5 g de N,N-diéthyl acéthylthio-2 propionamide(R) sous forme d'une huile légèrement jaune [P.E.$_{17\ Pa}$ = 105-107 ° C ; $[\alpha]_D^{20}$ = +156 ° (c = 18,1 ; $CHCl_3$)].

Le N,N-diétyl chloro-2 propionamide(S) peut être obtenu de la manière suivante :

A une solution de 175,2 g de chlorure de chloro-2 propionyle(S) dans 900 cm3 de chloroforme maintenue à 0 ° C on ajoute 480 cm3 de diéthylamine en 1 heure. Le mélange réactionnel est additionné de 400 cm3 d'eau distillée et de 300 cm3 de dichlorométhane. La phase organique est décantée puis lavée par 300 cm3 d'une solution aqueuse d'acide chlorhydrique 2N puis 400 cm3 d'eau distillée. La phase organique est séchée sur du sulfate de sodium, filtrée puis concentrée à sec sous pression réduite (2,7 kPa) à une température voisine de 30 ° C. Le résidu obtenu est distillé sous pression réduite (17 Pa). On obtient ainsi 161,9 g de N,N-diéthyl chloro-2 propionamide(S) sous forme d'une huile incolore [P.E.$_{17\ Pa}$ = 83-85 ° C ; $[\alpha]_D^{20}$ = +39,7 ° (c = 10,4 ; $CHCl_3$ )].

Les chlorures de chloro-2 propionyle(R) et (S) peuvent être préparés selon la méthode décrite par S-C.J. FU, S.M. BIRNBAUM et J.P. GREENSTEIN, J. Am. Chem. Soc., 76, 6054 (1954).

## EXEMPLE 4

A 2 g de [diéthylamino-1 propyl-2(S) thio-26 pristinamycine $II_B$ (isomère A) en solution dans 30 cm3 de méthanol on ajoute en 30 minutes à -60 ° C, 20 cm3 d'une solution aqueuse de 1,01 g d'Oxone®. La suspension obtenue est agitée 30 minutes à -60 ° C puis 16 heures à -20 ° C. On ajoute alors 0,2 g d'Oxone ® en solution dans 5 cm3 d'eau puis on agite 30 minutes à -20 ° C. Le mélange réactionnel est dilué par 100 cm3 d'eau, additonné de 0,5 g de noir NORIT SX ULTRA agité 15 minutes, filtré sur Celite puis rincé par 3 fois 5 cm3 d'eau. La phase aqueuse est extraite par 3 fois 50 cm3 d'acétate d'éthyle, ajustée à pH 7 par addition de bicarbonate de sodium solide, saturée en chlorure de sodium puis lavée par 3 fois 50 cm3 de dichlorométhane.

Les phases organiques sont rassemblées, séchées sur sulfate de magnésium, filtrées puis concentrées à sec sous pression réduite (2,7 kPa) à 30 ° C. On obtient ainsi une meringue blanche qui est agitée dans 20 cm3 d'éther diéthylique. Le solide résultant est filtré, puis séché sous pression réduite (270 Pa) à 20 ° C pour donner 1,5 g de [diéthylamino-1 propyl-2(S)] sulfinyl-26 pristinamycine $II_B$ sous forme d'un solide blanchâtre (isomère $A_2$ : 90 %, isomère $A_1$ : 5 %) fondant vers 130 ° C dont les caractérisitques RMN sont identiques à celles du produit obtenu ci-après à l'exemple 5.

La [diéthylamino-1 propyl-2(S)] thio-216 pristinamycine $II_B$ (isomère A) peut être obtenue de la façon suivante :

A 10,5 g de pristinamycine $II_A$ en solution dans 200 cm3 d'un mélange chlorure de méthylène-méthanol (50-50 en volumes), on ajoute à -20 ° C, sous azote, 3,2 g de diéthylamino-1 propanethiol-2(S). Après 70 heures d'agitation à -20 ° C, le mélange réactionnel est concentré à sec sous pression réduite (2,7 kPa) à 30 ° C puis repris par 100 cm3 d'acétate d'éthyle. La solution obtenue est lavée par 100 cm3 d'une solution

aqueuse 0,2N de sulfate acide de potassium. La phase aqueuse est décantée puis lavée par 3 fois 100 cm3 d'acétate d'éthyle. La phase organique est lavée par 50 cm3 d'eau distillée puis les phases aqueuses sont rassemblées, ajustées à pH 7 par addition de 2 g de bicarbonate de sodium solide et lavées par 100 cm3 puis 2 fois 50 cm3 de dichlorométhane. Ces dernières phases organiques sont rassemblées, séchées sur sulfate de sodium, filtrées puis concentrées à sec sous pression réduite (2,7 kPa) à 30 °C. Le solide obtenu est agité dans 150 cm3 d'éther éthylique, filtré puis rincé par 3 fois 10 cm3 d'éther. On obtient ainsi 9,2 g d'un solide beige qui est recristallisé dans 20 cm3 d'acétonitrile. Après filtration, séchage à 20 °C sous pression réduite (270 Pa) on obtient 2,3 g de [diéthylamino-1 propyl-2(S)] thio-26 pristinamycine $II_B$ - (isomère A) sous forme de cristaux blancs fondant vers 128 °C.

Spectre RMN

1,04 (t, $-N(CH_2CH_3)_2$)

1,39 (d, 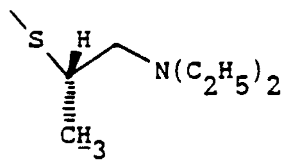

1,73 (s, $-CH_3$ en 33)

1,90 et 2,13 (2mt, $>CH_2$ en 25)

2,4 à 2,7 (mt, $-CH_2-N(CH_2CH_3)_2$)

2,78 (mt, H en 4)

2,93 et 3,12 (2dd, $>CH_2$ en 15)

3,02 (mt, 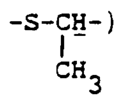

3,55 (mt, H en 26)

3,84 (s, $>CH_2$ en 17)

4,81 (s large, H en 27)

5,49 (d, H en 13)

6,15 (d, H en 11)

6,30 (m, $>NH$ en 8)

6,53 (dd, H en 5)

8,13 (s, H en 20)

Le diéthylamino-1 propanethiol-2(S) peut être préparé de la manière suivante :

A une suspension de 42,6 g d'hydrure de lithium et d'aluminium dans 1500 cm3 d'éther éthylique, on ajoute goutte à goutte en 1 heure et 20 minutes, 171 g de N,N-diéthyl mercapto-2 propionamide(S) en solution dans 500 cm3 d'éther éthylique. Le mélange réactionnel est ensuite maintenu 2 heures et 30 minutes au reflux puis refroidi à une température voisine de 0 °C. On additionne alors 49,8 cm3 d'eau distillée de manière que la température du mélange ne dépasse pas 20 °C, puis 36,6 cm3 de soude 5N, et 166 cm3 d'eau distillée. Le mélange réactionnel est filtré, le pH du filtrat est ajusté à 8 par addition de 70 cm3 d'acide acétique. Le mélange obtenu est à nouveau filtré, le filtrat est séché sur du sulfate de sodium, filtré puis concentré à sec sous pression réduite (2,7 kPa) à une température voisine de 30 °C. On obtient une huile jaune qui est purifiée par distillation sous pression réduite (1,3 kPa). On obtient ainsi 100 g de diéthylamino-1 propanethiol-2(S) sous forme d'une huile incolore [P.E.$_{1,3\ kPa}$ = 55-56 °C ; $[\alpha]_D^{20}$ = +39,6 ° (c = 5,6 $CH_3OH$)].

Le N,N-diéthyl mercapto-2 propionamide(S) peut être préparé de la manière suivante :

A 1100 cm3 d'une solution 5N de soude maintenue à 20 °C, on ajoute en 30 minutes, 223 g N,N-diéthyl acétylthio-2 propionamide(S) dans 1000 cm3 d'éther éthylique. Le mélange réactionnel est agité 20 heures à une température voisine de 20 °C. La phase aqueuse est séparée par décantation puis lavée par 3 fois 250 cm3 d'éther éthylique. Le pH de la phase aqueuse est ensuite ajusté à 5 par addition lente de 280 cm3 d'acide acétique. Le mélange obtenu est extrait par 500 cm3 puis 2 fois 250 cm3 de dichlorométhane, les phases organiques réunies sont séchées sur du sulfate de sodium en présence de noir de carbone, filtrées puis concentrées à sec sous pression réduite (2,7 kPa) à une température voisine de 30 °C. On

obtient ainsi 171,7 g de N,N-diéthyl mercapto-2 propionamide(S) sous forme d'une huile violacée ($[\alpha]_D^{20}$ = +20,7° (c = 3, $CH_3OH$)).

Le N,N-diéthyl acétylthio-2 propionamide(S) peut être préparé de la manière suivante :

A une suspension de 16 g de sel de potassium de l'acide thiolacétique dans 70 cm3 d'éthanol, on ajoute 20 g de N,N-diéthyl chloro-2 propionamide(R) en solution dans 30 cm3 d'éthanol. Le mélange réactionnel est ensuite chauffé à une température voisine de 55°C pendant 2 heures puis concentré à sec sous pression réduite (130 Pa) à une température voisine de 60°C. Le résidu obtenu est trituré avec 300 cm3 de dichlorométhane puis lavé avec 200 cm3 d'eau distillée. La phase organique est décantée, séchée sur du sulfate de sodium, filtrée puis concentrée à sec sous pression réduite (2,7 kPa) à une température voisine de 30°C. Le résidu obtenu est distillé sous pression réduite (17 Pa). On obtient ainsi 22 g de N,N-diéthyl acétylthio-2 propionamide(S) sous forme d'une huile légèrement jaune [$P.E._{17\ Pa}$ = 105-107°C ; $[\alpha]_D^{20}$ = -169° (c = 10, $CHCl_3$)].

Le N,N-diéthyl chloro-2 propionamide(R) peut être préparé de la manière suivante :

A une solution, maintenue à 20°C, de 87,7 g de chlorure de chloro-2 propionyle(R) dans 600 cm3 de chloroforme, on ajoute 153 g de diéthylamine en 1 heure. Le mélange réactionnel est lavé par 500 cm3 d'eau distillée, 3 fois 500 cm3 d'une solution aqueuse d'acide chlorhydrique 1N, puis 500 cm3 d'eau distillée. La phase organique est séchée sur du sulfate de sodium, filtrée puis concentrée à sec sous pression réduite (2,7 kPa) à une température voisine de 30°C. Le résidu obtenu est distillé sous pression réduite (17 Pa). On obtient ainsi 43 g de N,N-diéthyl chloro-2 propionamide(R) sous forme d'une huile incolore [$P.E._{17\ Pa}$ = 75-80°C ; $[\alpha]_D^{20}$ = -43,3° (c = 10 ; $CHCl_3$ )].

## EXEMPLE 5

A une suspension de 63 g de pristinamycine $II_A$ dans 630 cm3 de méthanol, on ajoute sous azote à -38°C et en 30 minutes, 17,6 g de diéthylamino-1 propanethiol-2 (S). Après 20 heures d'agitation on ajoute à -38°C, 150 cm3 d'eau distillée puis en 30 minutes, 410 cm3 d'une solution aqueuse de 33,6 g d'Oxane®. La suspension obtenue est agitée 1 heure à -38°C, additionnée de 1,2 g de thiosulfate de sodium, filtrée à 20°C sur verre fritté puis lavée par 3 fois 200 cm3 d'eau distillée. On ajoute à la phase aqueuse 20 g de noir NORIT SX ULTRA, agite 30 minutes puis filtre sur Célite en lavant le filtrat par 3 fois 200 cm3 d'eau distillée. La solution est ajustée à pH 7 avec 15 g bicarbonate de sodium solide puis lavée par 3 fois 500 cm3 de dichlorométhane. Les phases organiques sont rassemblées, séchées sur sulfate de sodium, filtrées puis concentrées à sec sous pression réduite (2,7 kPa) à 30°C. On obtient 55 g d'une meringue beige qui est agitée dans 500 cm3 d'éther éthylique. Après filtration, lavage par 3 fois 50 cm3 d'éther éthylique puis séchage sous vide partiel (270 Pa) à 20°C, on obtient 46,6 g d'un solide blanc qui est dissous dans 150 cm3 d'un mélange dichlorométhane-méthanol (98-2 en volumes) puis additonné de 4 cm3 d'acide acétique. Cette solution est purifiée par chromatographie flash [éluant : dichlorométhane-méthanol (98-2 en volume)] en recueillant des fractions de 100 cm3. Après concentration sous pression réduite (2,7 kPa) à 30°C, on obtient 36 g d'un solide qui est dissous dans 400 cm3 d'eau distillée additionnée de 6,5 g de sulfate acide de potassium. La solution est lavée par 4 fois 500 cm3 d'acétate d'éthyle, ajustée à pH 7 par addition de 5 g de bicarbonate de sodium solide puis extraite par 200 cm3 puis 2 fois 150 cm3 de dichlorométhane. Les phases organiques sont réunies, séchées sur sulfate de magnésium, filtrées puis concentrées à sec sous pression réduite (2,7 kPa) à 30°C pour donner 33,8 g d'une meringue beige qui est agitée dans 300 cm3 d'éther éthylique. Après filtration, on isole 29,3 g de [diéthylamino-1 propyl-2(S)] sulfinyl-26 pristinamycine $II_B$ (isomère $A_2$) sous forme d'une poudre jaune pâle fondant vers 140°C.

Spectre RMN (isomère $A_2$)

1,05 (t, $-N(CH_2-\underline{CH_3})_2$)

1,27 (d, 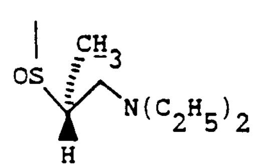 )

1,74 (s, $-CH_3$ en 33)

2,10 (mt, 1H du $>CH_2$ en 25)

2,45 à 2,70 (mt, 1H du $>CH_2$ en 25, 1H du $>\underline{CH_2}-N(CH_2CH_3)_2$, $-N(\underline{CH_2}CH_3)_2$)

2,80 (mt, H en 4)

2,88 (mt, 1H du > $CH_2$-$N(CH_2CH_3)_2$)
2,90 et 3,15 (2dd, > $CH_2$ en 15)

3,06 (mt, 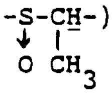)

3,41 (mt, H en 26)
3,82 (s, > $CH_2$ en 17)
4,77 (s large, H en 27)
5,50 (d, H en 13)
6,20 (d, H en 11)
6,55 (mt, > NH en 8)
6,63 (dd, H en 5)
8,11 (s, H en 20)

EXEMPLE 6

A 50 g de (diéthylamino-2 éthyl) thio-26 pristinamycine $II_B$ en solution dans 200 cm3 de dichlorométhane et 250 cm3 d'eau distillée, on ajoute à 0 °C, en 20 minutes, 30,7 g d'Oxone® en solution dans 140 cm3 d'eau distillée. Après 10 minutes d'agitation, la phase organique est décantée, la phase aqueuse est lavée par 100 cm3 de dichlorométhane puis ajustée à pH 7 par addition de 120 cm3 d'une solution saturée de bicarbonate de sodium. La phase aqueuse est lavée par 5 fois 100 cm3 de dichlorométhane en ajustant le pH à 7, à chaque lavage par addition de bicarbonate de sodium. Les phases organiques sont réunies, séchées sur sulfate de magnésium, filtrées puis concentrées à sec sous pression réduite (2,7 kPa) à 40 °C. On obtient ainsi 31 g de (diéthylamino-2 éthyl) sulfinyl-26 pristinamycine $II_B$ (isomère $A_2$ : 85 %, isomère $A_1$ : 10 %) sous forme d'une poudre beige clair dont les caractérisitiques sont identiques à celles du produit obtenu à l'exemple 1.

EXEMPLE 7

A 2 g de [diéthylamino-1 propyl-2(S)] thio-26 pristinamycine $II_B$ (isomère A) en solution dans 30 cm3 d'éthanol et 20 cm3 d'eau, on ajoute lentement à 0 °C 0,08 cm3 d'acide sulfurique concentré puis 0,9 g d'Oxone® en solution dans 10 cm3 d'eau distillée. La solution obtenue est agitée pendant 4 heures à 20 °C. On ajoute ensuite du bicarbonate de sodium solide jusqu'à pH 4 et l'agitation est laissée 2 jours à 20 °C. On ajoute alors 25 cm3 d'eau distillée, 2 g de noir NORIT SX ULTRA. Le mélange est filtré sur Célite, rincé avec 20 cm3 de dichlorométhane puis ajusté à pH 7 avec du bicarbonate de sodium solide.

La phase aqueuse est décantée puis lavée par 2 fois 25 cm3 de dichlorométhane. Les phases organiques sont séchées sur sulfate de magnésium, filtrées puis concentrées à sec sous pression réduite (2,7 kPa) à 40 °C.

On obtient ainsi 1,6 g d'un solide beige contenant 80 % de [diéthylamino-1 propyl-2(S)] sulfinyl-26 pristinamycine $II_B$ (isomère $A_2$), 10 % de l'isomère $A_1$ et 10 % de sulfure de départ et dont les caractéristiques sont identiques à celles décrites à l'exemple 4.

EXEMPLE 8

A 6g de [diéthylamino-1 propyl-2(S)] thio-26 pristinamycine $II_B$ (isomère A) en solution dans 20 cm3 d'eau distillée et 90 cm3 de méthanol placé à -30 °C, on ajoute 0,24 cm3 d'acide sulfurique concentré puis lentement 2,7 g d'Oxone® en solution dans 10 cm3 d'eau distillée. La solution trouble obtenue est agitée à -30 °C pendant 1 heure. On ajoute alors 0,6 g de noir NORIT SX ULTRA puis le mélange est filtré sur Célite. Le filtrat est ajusté à pH 7 par addition de bicarbonate de sodium solide puis lavé par 3 fois 50 cm3 de dichlorométhane. Les phases organiques sont rassemblées, séchées sur sulfate de magnésiuum, filtrées puis concentrées à sec sous pression réduite (2,7 kPa) à 30 °C.

Le solide résultant est agité dans un mélange de 20 cm3 d'acétate d'éthyle et de 70 cm3 d'éther éthylique, filtré puis lavé par 50 cm3 d'éther éthylique. On obtient ainsi 4,6 g d'un solide beige clair qui est purifié par chromatographie flash [éluant : acétate d'éthyle-méthanol (90-10 en volumes)] en recueillant des fractions de 15 cm3. Après concentration à sec des fractions 47 à 54 sous pression réduite (2,7 kPa) à

30 ° C on obtient 1,7 g de [diéthylamino-1 propyl-2(s)] sulfinyl-26 pristinamycine $II_B$ (isomère $A_2$) contenant 10 % de sulfure de départ et ayant des caractéristiques identiques au produit décrit à l'exemple 4.

EXEMPLE 9

A 25,5 g de [diéthylamino-2 propyl(2S)] thio-26 pristinamycine $II_B$ en solution dans 350 cm3 d'éthanol on ajoute à -30 ° C, 200 cm3 d'eau distillée, puis lentement 15,1 g d'Oxone® en solution dans 70 cm3 d'eau distillée. Après 1 heure 30 minutes d'agitation à -30 ° C on ajoute 3,7 g de thiosulfate de sodium en solution dans 20 cm3 d'eau. Le mélange réactionnel est ensuite versé sur 400 cm3 d'eau distillée et 200 cm3 de dichlorométhane, ajusté à pH 7 avec du bicarbonate de sodium. La phase organique est décantée et la phase aqueuse lavée par 3 fois 200 cm3 de dichlorométhane. Les phases organiques sont réunies, séchées sur sulfate de magnésium, filtrées, puis concentrées à sec sous pression réduite (2,7 kPa) à 30 ° C. On obtient ainsi 24 g d'un solide jaune clair qui est purifié par chromatographie flash [éluant : chloroforme-méthanol (90-10 en volumes)]. Après concentration à sec sous pression réduite (2,7 kPa) à 30 ° C des fractions 23 à 28 (volume 50 cm3) on obtient une meringue jaune qui est agitée dans 100 cm3 d'éther éthylique, filtrée puis séchée sous pression réduite (90 Pa) à 20 ° C. On obtient ainsi 11,3 g de [diéthylamino-2 propyl(2S)] sulfinyl-26 pristinamycine $II_B$ (isomère $A_2$) sous forme de poudre jaune clair fondant vers 128 ° C.
Spectre RMN

1,1 (mt, $-CH_2CH_3$ et 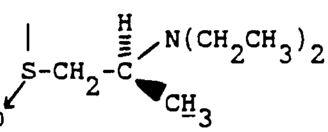 )

1,77 (s, $-CH_3$ en 33)
2 (mt, 1H de $-CH_2-$ en 25)

2,30 à 2,7 (mt, 1H de $-CH_2-$ en 25, 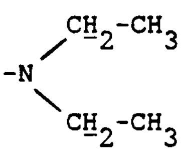 ,
1H de $-SO-CH_2-$)

2,97 (mt, 1H du $-SO-CH_2-$)
2,78 (mt, > CH- en 4)
2,92 et 3,10 (2dd, $-CH_2-$ en 15)
3,13 (mt, > CH- en 26)
3,50 (mt, > N-CH-)
3,81 (s, $CH_2-$ en 17)
4,77 (d, > CH- en 27)
5,52 (d, =CH- en 13)
6,18 (d, =CH- en 11)
6,48 (mt, -NH- en 8)
6,57 (dd, =CH- en 5)
8,12 (s, =CH- en 20)

La [diéthylamino-2 propyl(2S)] thio-26 pristinamycine $II_B$ (isomère A) peut être obtenue de la manière suivante :

A 31,5 g de pristinamycine $II_A$ en solution dans 230 cm3 de méthanol et 70 cm3 de chloroforme placée sous atmosphère d'azote on ajoute à -40 ° C 10 g de diéthylamino-2 propanethiol (S) en solution dans 50 cm3 de chloroforme. Après 4 jours d'agitation à -40 ° C, le mélange est concentré à sec sous pression réduite (2,7 kPa) à 35 ° C puis le solide résultant est agité dans 500 cm3 d'éther éthylique, filtré puis à nouveau agité dans 300 cm3 d'éther diéthylique. Après filtration, le solide est séché (90 Pa) à 20 ° C puis purifié par chromatographie flash [éluant : chloroforme-méthanol (90-10 en volumes)] en recueillant des fractions de 100 cm3. Après concentration à sec sous pression réduite (2,7 kPa) à 30 ° C des fractions 18 à 30 on obtient 26,6 g de [diéthylamino-2 propyl(2S)] thio-26 pristinamycine $II_B$ (isomère A) sous forme d'un

solide jaune clair fondant vers 110 ° C.
Spectre RMN :

1,05 (mt, $CH_3$-$CH_2$- et -S-$CH_2$- 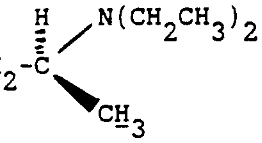 )

1,72 (s, -$CH_3$ en 33)
1,95 à 2,10 (mt, -$CH_2$- en 25)

2,50 (mt, - 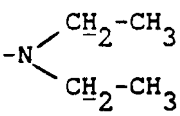 )

2,60, 2,87 et 3,04 (mt, -S-$CH_2$-CH < )
2,77 (mt, > CH- en 4)
2,9 et 3,10 (2dd, -$CH_2$- en 15)
3,35 (mt, > CH- en 26)
3,82 (s, -$CH_2$- en 17)
4,7 (d, > CH- en 27)
5,47 (d, =CH- en 13)
6,15 (d, =CH- en 11)
6,46 (m, -NH- en 8)
6,54 (dd, =CH- en 5)

Le diéthylamino-2 propanethiol(S) peut être préparé de la manière suivante :

A 39 g de dichlorhydrate de diéthylamino-2 propylisothiouronium (S) dans 140 cm3 d'éther éthylique on ajoute 59,4 cm3 d'une solution aqueuse de soude 5N. Après 30 minutes d'agitation sous atmosphère d'azote, la phase aqueuse est décantée puis lavée par 3 fois 150 cm3 d'éther éthylique. Les phases organiques sont rassemblées, séchées sur sulfate de magnésium, filtrées puis concentrées à sec sous pression réduite (2,7 kPa) à 30 ° C. Le liquide obtenu est distillé à 78 ° C sous 2,7 kPa pour donner 10,6 g de diéthylamino-2 propanethiol(S) sous la forme d'un liquide incolore (contenant 6 % de diéthylamino-1 propanethiol).

$[\alpha]_D^{20}$ = +32 ± 0,6 (c = 0,943 ; éthanol).

Le dichlorhydrate de diéthylamino-2 propylisothiouronium (S) peut être préparé de la manière suivante :

A 18,2 g de thiourée en solution dans 80 cm3 de N,N diméthylformamide on ajoute à 130 ° C une solution de 45 g de chlorohydrate de diéthylamino-2 chloro-1 propane(S) dans 150 cm3 de N,N diméthylformamide. Après 5 minutes à 130 ° C la solution est refroidie. Les cristaux obtenus sont filtrés, lavés à l'éther éthylique puis séchés sous pression réduite (90 Pa) à 20 ° C. On obtient ainsi 39 g de dichlorhydrate de diéthylamino-2 propylisothiouronium (S) sous forme de cristaux blancs fondant à 209 ° C.

$[\alpha]_D^{20}$ = -2,6 (c = 1 ; éthanol).

Le chlorhydrate de diéthylamino-2 chloro-1 propane(S) peut être préparé de la manière suivante :

A 110 cm3 de chlorure de thionyle on ajoute à 2 ° C, 50 g de chlorhydrate de diéthylamino-2 propanol-(S) par petites fractions. La solution obtenue est ensuite chauffée pendant 4 heures à 60 ° C. Le chlorure de thionyle en excès est éliminé par distillation sous pression réduite (90 kPa) puis on ajoute 250 cm3 d'éther éthylique au résidu obtenu. Le solide résultant est filtré, rincé par 300 cm3 d'éther éthylique puis recristallisé dans 150 cm3 de méthylisobutylcétone. Après filtration, lavage à l'éther éthylique, on obtient 45,6 g de chlorohydrate de diéthylamino-2 chloro-1 propane(S) sous forme de cristaux blanc fondant à 103 ° C.

$[\alpha]_D^{20}$ = 0 (c = 0,7 ; éthanol).

Le chlorhydrate de diéthylamino-2 propanol(S) peut être préparé de la manière suivante :

A une suspension de 18,23 g d'hydrure de litium et d'aluminium dans 2000 cm3 de tétrahydrofuranne, on ajoute en 20 minutes 48,66 g de N-acétyl N-éthyl-amino-2 propanol(S) en solution dans 300 cm3 de tétrahydrofuranne. Le mélange réactionnel est ensuite maintenu 4 heures au reflux puis refroidi à 0 ° C. On additionne alors lentement 22 cm3 d'eau distillée puis 16 cm3 de soude aqueuse 5N puis 72 cm3 d'eau distillée. Après 1 heure d'agitation à 20 ° C le mélange est filtré sur Célite. Le filtrat est concentré à sec

sous pression réduite (2,7 kPa) à 30 ° C, repris par 300 cm3 de dichlorométhane, séché sur sulfate de magnésium, filtré puis concentré à sec sous pression réduite (2,7 kPa) à 30 ° C pour donner 42,45 g d'un liquide jaune clair qui est distillé sous pression réduite. On obtient ainsi 26,56 g de diéthylamino-2 propanol-(S) sous forme d'un liquide incolore. [P.E.$_{1\ kPa}$ = 55,5 ° C].

On obtient 31,41 g de chlorhydrate de diéthylamino-2 propanol(S) sous forme d'un solide blanc fondant à 98 ° C après filtration des cristaux obtenus en ajoutant 40,5 cm3 d'une solution 4,9N d'acide chlorhydrique dans l'éther éthylique au produit précédent en solution dans 136 cm3 d'acétone.

$[\alpha]_D^{20}$ = +20,7 (c = 1 ; éthanol).

Le N-acétyl N-éthyl-amino-2 propanol(S) peut être obtenu de la manière suivante :

On ajoute à 9,15 g de N-éthylamino-2 propanol(S) en solution dans 100 cm3 de dichlorométhane, 13,7 cm3 de triéthylamine à 0 ° C puis on ajoute en 45 minutes 7,1 cm3 de chlorure d'acétyle. Une fois l'addition terminée on laisse remonter la température à 20 ° C. Le mélange réactionel est dilué par 100 cm3 d'eau, ajusté à pH 9 par addition de carbonate de sodium puis lavé par 2 fois 200 cm3 de dichlorométhane. Les phases organiques sont rassemblées, séchées sur sulfate de magnésium, filtrées puis concentrées à sec sous pression réduite (2,7 kPa) à 30 ° C pour donner 9,54 g d'un liquide jaune qui est distillé à 108 ± 2 ° C sous pression réduite (26,3 Pa). On obtient ainsi 6 g d'un liquide incolore contenant environ 80 % de N-acétyl N-éthyl-amino-2 propanol(S) et 20% du dérivé diacétylé correspondant (utilisé tel quel dans la suite de la synthèse).

Le N-éthylamino-2 propanol(S) peut être préparé de la manière suivante :

A une suspension de 81 g d'hydrure de lithium et d'aluminium dans 4000 cm3 de tétrahydrofuranne, on ajoute à 0 ° C 227 g d'acétylamino-2 propionate d'éthyle(S) dans 800 cm3 de tétrahydrofuranne en 45 minutes. Une fois l'addition terminée la température est remontée à 20 ° C puis le mélange chauffé au reflux pendant 5 heures. Après refroidissement à 0 ° C on additionne lentement 97 cm3 d'eau distillée puis 72 cm3 d'une solution aqueuse de soude 5N puis enfin 320 cm3 d'eau distillée. le mélange est filtré à 20 ° C sur Célite. Le filrat est concentré à sec sous pression réduite (2,7 kPa) à 30 ° C, repris par 500 cm3 de dichlorométhane, séché sur sulfate de magnésium, filtré puis concentré à sec sous pression réduite (2,7 kPa) à 30 ° C pour donner 143 g d'un liquide jaune. Après distillation sous pression réduite (460 Pa) à 57 ° C, on obtient 90,8 g de N-éthylamino-2 propanol(S) sous forme d'un liquide incolore.

$[\alpha]_D^{20}$ = +45,4 (c = 1,48 ; éthanol).

L'acétylamino-2 propionate d'éthyle peut être préparé comme décrit par J.P. WOLFF III et coll., Biochemistry 2, 493 (1963).

**Revendications**

**1.** Un procédé de préparation d'un nouveau dérivé de la pristinamycine $II_B$, de formule générale :

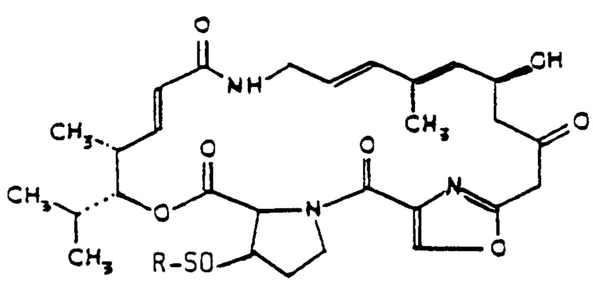

(I)

dans laquelle le symbole R représente

- soit un radical hétérocyclyle azoté de 4 à 7 chaînons, contenant éventuellement 1 ou plusieurs autres hétéroatomes choisis parmi l'azote, l'oxygène ou le soufre à l'état de sulfoxyde ou de sulfone, et éventuellement substitué par un radical alcoyle,
- soit une chaîne alcoyle contenant 2 à 4 atomes de carbone substituée par 1 ou 2 radicaux choisis parmi phényle, cycloalcoylamino ou N-alcoyl N-cycloalcoyl-amino contenant 3 à 6 chaînons, alcoylamino, dialcoylamino ou dialcoylcarbamoyloxy (les parties alcoyle de ces 2 derniers radicaux pouvant éventuellement former avec l'atome d'azote auquel elles sont rattachées un hétérocycle saturé ou insaturé de 4 à 7 chaînons contenant éventuellement un autre hétéroatome choisi parmi l'azote, l'oxygène ou le soufre à l'état de sulfoxyde ou de sulfone, et éventuellement substitué par un radical alcoyle), ou substituée par un ou plusieurs hététrocycles azotés de 4 à 7

chaînons contenant éventuellement 1 ou 2 autres hétéroatomes choisis parmi l'azote, l'oxygène ou le soufre à l'état de sulfoxyde ou de sulfone, et éventuellement substitués par un radical alcoyle, lesdits hétérocycles étant rattachés à la chaîne qui les porte par l'intermédiaire d'un atome de carbone, étant entendu que l'un au moins des substituants portés par la chaîne alcoyle ci-dessus est un substituant azoté capable de former des sels,

- soit un radical [méthyl-1 pyrrolidinyle-2(S)] méthyl, et sauf mention spéciale, les radicaux alcoyle cités ci-dessus sont droits ou ramifiés et contiennent 1 à 10 atomes de carbone, sous ses formes isomères ou leurs mélanges, ainsi que ses sels d'addition avec les acides, caractérisé en ce que l'on oxyde au moyen de monopersulfate de potassium un dérivé de la pristinamycine $II_B$ (son sel ou un dérivé protégé) de formule générale :

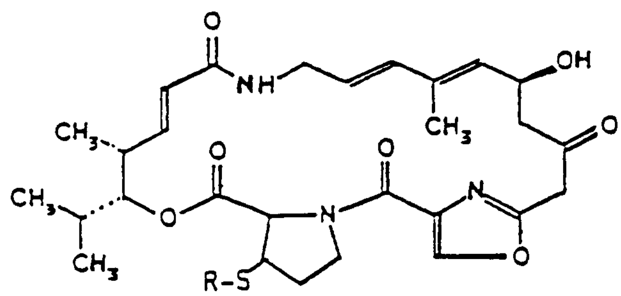

(II)

dans laquelle R est défini comme précédemment, étant entendu que dans le cas où R contient un hétérocycle soufré l'atome de soufre peut se trouver à l'état de sulfure, de sulfoxyde ou de sulfone, puis sépare éventuellement le produit obtenu en ses isomères, le cas échéant élimine le radical protecteur et transforme éventuellement le produit obtenu en un sel d'addition avec un acide.

## Claims

**1.** A process for the preparation of a new pristinamycin $II_B$ derivative of general formula:

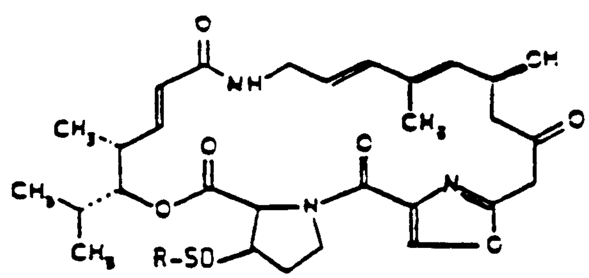

(I)

in which the symbol R represents

either a 4- to 7-membered nitrogen-containing heterocyclic radical, optionally containing 1 or more other hetero atoms chosen from amongst nitrogen, oxygen or sulphur in the sulphoxide or the sulphone form, and optionally substituted with an alkyl radical,

or on alkyl chain containing 2 to 4 carbon atoms, substituted with 1 or 2 radicals chosen from amongst phenyl, cycloalkylamino or 3- to 6-membered N-alkyl-N-cycloalkylamino, alkylamino, dialkylamino or dialkylcarbamoyloxy (it being possible for the alkyl parts of these last 2 radicals optionally to form, with the nitrogen atom to which they are attached, a 4- to 7-membered saturated or unsaturated heterocycle optionally containing another hetero atom chosen from amongst nitrogen, oxygen or sulphur in the sulphoxide or the sulphone form, and optionally substituted with an alkyl radical) radicals or substituted with one or more 4- to 7-membered nitrogen-containing heterocycles optionally containing 1 or 2 other hetero atoms chosen from amongst nitrogen, oxygen or sulphur in the sulphoxide or the sulphone form and optionally substituted with an alkyl radical, the said heterocycles being attached to the chain which carries them via a carbon atom, it being understood that at least one

of the substituents carried by the alkyl chain above is a nitrogen-containing substituent capable of forming salts,

or a [(S)-1-methyl-2-pyrrolidinyl]methyl radical, and, unless otherwise stated, the alkyl radicals mentioned above are straight-chain or branched and contain 1 to 10 carbon atoms, in the isomeric forms thereof or the mixtures thereof and the addition salts thereof with acids, wherein

a pristinamycin $II_B$ derivative (a salt thereof or a protected derivative) of general formula:

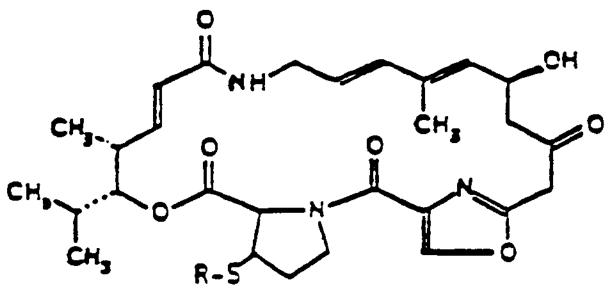

(II)

in which R is defined as above, it being understood that in the case where R contains a sulphur-containing heterocycle, the sulphur atom may be in the form of sulphide, sulphoxide or sulphone, is oxidised using potassium peroxymonosulphate, and the product obtained is separated into its isomers if required and, when appropriate, the protective radical is removed and the product obtained is converted into an addition salt with an acid if required.

## Patentansprüche

**1.** Verfahren zur Herstellung eines neuen Derivats von Pristinamycin $II_B$ der allgemeinen Formel:

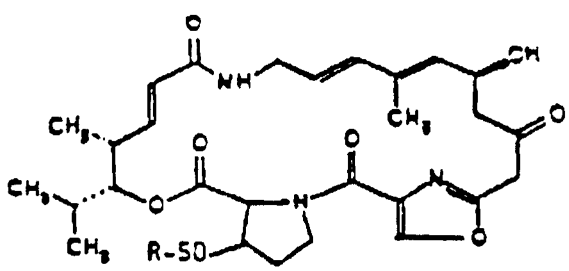

(I)

in welcher das Symbol R darstellt

- entweder einen stickstoffhaltigen Heterocyclylrest mit 4 bis 7 Gliedern, die gegebenenfalls 1 oder mehrere andere Heteroatome, ausgewählt aus Stickstoff, Sauerstoff oder Schwefel im Zustand des Sulfoxids oder Sulfons, enthält und gegebenenfalls durch einen Alkylrest substituiert ist,
- oder eine Alkylkette mit 2 bis 4 Kohlenstoffatomen, substituiert durch 1 oder 2 Reste, ausgewählt aus Phenyl-, Cycloalkylamino oder N-Alkyl-N-cycloalkyl-amino mit 3 bis 6 Gliedern, Alkylamino, Dialkylamino oder Dialkylcarbamoyloxy (die Alkylteile dieser zwei zuletztgenannten Reste können gegebenenfalls mit dem Stickstoffatom, an das sie gebunden sind, einen gesättigten oder ungesättigten Heterocyclus mit 4 bis 7 Gliedern bilden, der gegebenenfalls ein weiteres Heteroatom, ausgewählt aus Stickstoff, Sauerstoff oder Schwefel im Zustand des Sulfoxids oder Sulfons, enthält und gegebenenfalls durch einen Alkylrest substituiert ist), oder substituiert durch einen oder mehrere stickstoffhaltige Heterocyclen mit 4 bis 7 Gliedern, die gegebenenfalls 1 oder 2 andere Heteroatome, ausgewählt aus Stickstoff, Sauerstoff oder Schwefel im Zustand des Sulfoxids oder Sulfons, enthalten und gegebenenfalls durch einen Alkylrest substituiert sind, welche Heterocyclen an die sie tragende Kette über ein Kohlenstoffatom gebunden sind, wobei selbstverständlich zumindest der eine von der obigen Alkylkette getragene Substituent ein stickstoffhaltiger Substituent ist, der Salze zu bilden vermag,
- oder einen [1-Methyl-2(S)-pyrrolidinyl]-methylrest, und ohne speziellen Hinweis die oben genannten Alkylreste geradkettig oder verzweigt sind und 1 bis 10 Kohlenstoffatome enthalten, in seinen

isomeren Formen oder deren Mischungen, sowie seiner Säureadditionssalze, dadurch gekennzeichnet, daß man ein Derivat von Pristinamycin $II_B$ (sein Salz oder ein geschütztes Derivat) der allgemeinen Formel:

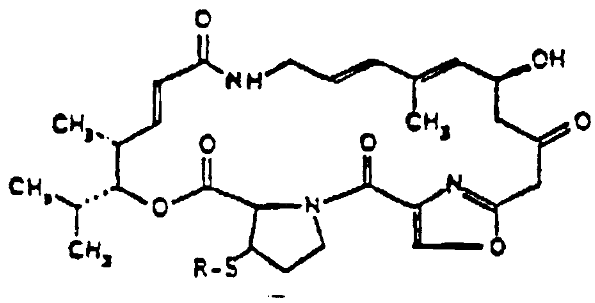

(II)

worin R die obige Bedeutung hat, wobei sich selbstverständlich, falls R einen schwefelhaltigen Heterocyclus enthält, das Schwefelatom im Zustand von Schwefel, Sulfoxid oder Sulfon befinden kann, mittels Kaliummonopersulfat oxidiert, dann gegebenenfalls das erhaltene Produkt in seine Isomeren spaltet, zutreffendenfalls die Schutzgruppe entfernt und das erhaltene Produkt gegebenenfalls in ein Additionssalz mit einer Säure überführt.